(19) Europäisches Patentamt
European Patent Office
Office européen des brevets

(11) **EP 4 243 012 A1**

(12) **DEMANDE DE BREVET EUROPEEN**

(43) Date de publication:
**13.09.2023 Bulletin 2023/37**

(21) Numéro de dépôt: **22315055.8**

(22) Date de dépôt: **10.03.2022**

(51) Classification Internationale des Brevets (IPC):
**G10L 15/22** (2006.01)    **G10L 21/0208** (2013.01)
**G10L 21/0216** (2013.01)    **A61B 5/00** (2006.01)
**G16H 80/00** (2018.01)

(52) Classification Coopérative des Brevets (CPC):
**G16H 40/67; A61B 5/746; G10L 15/22;**
**G16H 80/00;** G10L 21/0232

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**
Etats d'extension désignés:
**BA ME**
Etats de validation désignés:
**KH MA MD TN**

(71) Demandeur: **Tuito**
**13600 La Ciotat (FR)**

(72) Inventeurs:
• **Molac, M. Laurent**
**13600 LA CIOTAT (FR)**
• **Hafsati, M. Mohammed**
**13600 LA CIOTAT (FR)**

(74) Mandataire: **Fache, Sébastien**
**ETNA**
**3, rue Geoffroy Marie**
**75009 Paris (FR)**

(54) **SYSTEME ET PROCEDE D'AVERTISSEMENT ET DE COMMANDE PAR RECONNAISSANCE VOCALE**

(57)    L'invention porte principalement sur un système de commande vocale (1) comprenant au moins :
• Un premier dispositif de commande vocale (2) comportant des moyens d'acquisition d'un signal sonore et un second dispositif de commande vocale (3) comportant des moyens d'émission d'un signal d'avertissement,
• Des moyens de détection et de localisation (5) d'un utilisateur récepteur, et
• Un serveur (4) relié aux dispositifs de commande vocale (2, 3) et aux moyens de détection et de localisation (5).

Fig. 1

**Description**

L'invention s'inscrit dans le domaine de la commande d'appareils par la voix d'un utilisateur.

L'invention porte plus particulièrement sur un système et un procédé de commande par reconnaissance vocale.

La technologie de reconnaissance vocale est bien connue en soi, et repose sur la reconnaissance automatique de la voix humaine pour retranscrire un son sous la forme d'un texte, d'un signal sonore à émettre ou d'un signal de commande permettant de piloter un appareil équipé de cette technologie. La domotique est un parfait exemple de l'utilisation des commandes vocales pour piloter des appareils électriques.

Cependant, l'obtention d'un système mettant en œuvre un procédé de reconnaissance vocale fiable est délicate, car il est parfois difficile de transmettre efficacement une commande vocale au destinataire ou à l'appareil considérés.

En outre, cette fiabilité dépend d'un nombre important de paramètres liés à la qualité du son émis, de l'enregistrement sonore, du traitement du signal sonore et de la transduction du signal sonore en signal de commande. Chacune de ces étapes est susceptible d'avoir un impact négatif sur la qualité du signal sonore et/ou de commande, et donc sur la fiabilité de la commande par reconnaissance vocale.

Pour pallier les différents inconvénients évoqués plus haut, l'invention a pour objet un système et un procédé de commande par reconnaissance vocale à la fiabilité améliorée.

À cet effet, l'invention vise un système de commande vocale comprenant au moins :

- Un premier dispositif de commande vocale comportant des moyens d'acquisition d'un signal sonore et un second dispositif de commande vocale comportant des moyens d'émission d'un signal d'avertissement, et
- Un serveur relié aux dispositifs de commande vocale,

Lequel système de commande vocale est configuré pour :

- Acquérir un signal sonore via le premier dispositif de commande vocale, au moins une partie dudit signal sonore émis par un utilisateur émetteur se situant dans une plage de fréquences dites vocales comprise entre 50 et 500 Hz, et
- Emettre un signal d'avertissement à destination de l'utilisateur récepteur via le second dispositif de commande vocale.

Le système de commande peut également comporter les caractéristiques optionnelles suivantes considérées isolément ou selon toutes les combinaisons techniques possibles :

- Le système comprend des moyens de détection et de localisation d'un utilisateur récepteur relié au serveur, lequel système est configuré pour :

  • Suite à l'acquisition du signal sonore, détecter et localiser un utilisateur récepteur via les moyens de détection et de localisation, et
  • Préalablement à l'émission du signal d'avertissement, identifier le second dispositif de commande vocale le plus proche de la position de l'utilisateur récepteur détecté.

- Le système comprend au moins un dispositif d'identification personnalisé prévu pour être associé à l'utilisateur récepteur considéré.
- Chaque dispositif de commande vocale est apte à commander au moins un appareil électrique et comprend un espace de stockage comportant une base de données de caractéristiques représentatives de commandes vocales de référence, chaque commande vocale correspondant à un signal logique de commande de l'appareil électrique, et des moyens informatiques aptes à :

  • Extraire un signal vocal à partir du signal sonore détecté ;
  • Extraire au moins une caractéristique du signal vocal ;
  • Comparer la caractéristique du signal vocal avec la base de données de caractéristiques représentatives de commandes vocales de référence, et
  • Identifier le signal logique de commande de l'appareil électrique.

- Le serveur comprend une base de données de caractéristiques représentatives de commandes vocales associées à plusieurs appareils électriques considérés, lequel serveur est apte à :

  • Comparer la caractéristique de la commande vocale avec la base de données de caractéristiques représentatives des commandes vocales associées à plusieurs appareils électriques considérés ;
  • Associer la commande vocale reçue avec l'appareil électrique à commander, et
  • Envoyer au dispositif de commande vocale une information indiquant l'appareil électrique identifié devant être commandé.

- Le système comprend au moins un autre serveur dit serveur tiers, connecté au serveur et comprenant une base de données de caractéristiques représentatives d'un signal vocal de référence, le système étant apte à :

- Associer le signal vocal reçu avec une requête informatique d'interrogation de la base de données résidant sur le serveur tiers,
- Recevoir de la part du serveur tiers la réponse à la requête informatique d'interrogation de la base de données, et
- Envoyer au dispositif de commande vocale considéré les caractéristiques représentatives du signal vocal de référence associées à la réponse du serveur tiers.

L'invention vise également un procédé d'avertissement par commande vocale à l'aide d'un système de commande vocale comprenant au moins un premier dispositif de commande vocale comportant des moyens de réception d'un signal sonore, un second dispositif de commande vocale comportant des moyens d'émission d'un signal d'avertissement, des moyens de détection et de localisation d'un utilisateur récepteur et un serveur relié aux dispositifs de commande vocale et aux moyens de détection et de localisation, lequel procédé comprend au moins les étapes successives de :

- Détection par le premier dispositif de commande vocale d'un signal sonore dont au moins une partie émise par un utilisateur émetteur se situe dans une plage de fréquences dites vocales comprise entre 50 et 500 Hz, et
- Emission d'un signal d'avertissement à destination de l'utilisateur récepteur via le second dispositif de commande vocale.

Le procédé d'avertissement peut également comporter les caractéristiques optionnelles suivantes considérées isolément ou selon toutes les combinaisons techniques possibles :

- Le procédé comprend en outre les étapes suivantes :

  - Suite à l'étape de détection du signal sonore, détection et localisation d'un utilisateur récepteur, et
  - Préalablement à l'étape d'émission du signal d'avertissement, identification du second dispositif de commande (3) le plus proche de la position de l'utilisateur récepteur détecté.

- Le premier dispositif de commande vocale et/ou le serveur comprennent un espace de stockage comportant une base de données de caractéristiques de signaux vocaux de référence chacun correspondant à un signal d'avertissement vocal déterminé, lequel procédé comprend, préalablement à l'étape de détection et de localisation d'un utilisateur récepteur, les étapes successives de :

  - Extraction d'un signal vocal à partir du signal sonore détecté et d'au moins une caractéristique représentative de ce signal vocal ;
  - Comparaison de la caractéristique du signal vocal avec la base de données de caractéristiques représentatives des signaux vocaux de référence, et
  - Identification du signal d'avertissement vocal devant être émis par le second dispositif de commande vocale:

- Une analyse spatiale du signal sonore mise en œuvre par le procédé identifie au moins deux signaux vocaux, lequel procédé comprend les étapes successives de :

  - Détermination d'une pluralité de directions couvrant le spectre spatial complet autour des moyens d'acquisition du dispositif de commande vocale ;
  - Détermination des caractéristiques spectrales du signal sonore associé à chaque direction ;
  - Détermination des directions recevant des signaux sonores dont la puissance acoustique est supérieure à un seuil déterminé,
  - Association de chaque signal sonore dépassant le seuil déterminé à une source sonore,
  - Détermination du nombre de sources sonores et association de chaque source à la direction considérée, et
  - Amélioration des caractéristiques spectrales de chaque source sonore identifiée en soustrayant à chacune de ces sources sonores les caractéristiques spectrales des autres sources identifiées.

- Le procédé comprend une étape d'identification d'au moins une caractéristique correspondant à des altérations du signal vocal et d'association de chaque signal vocal altéré avec un signal vocal intact de référence correspondant par comparaison de leurs caractéristiques et en associant les caractéristiques les plus proches.
- Le procédé comprend une étape d'identification d'au moins une caractéristique correspondant à des altérations du signal vocal et d'association de chaque signal vocal altéré avec un signal vocal intact de référence correspondant par comparaison de leurs caractéristiques et en associant les caractéristiques les plus proches
- Le procédé comprend une étape préalable d'enrichissement de la base de données de signaux altérés avec les signaux vocaux de référence modifiés par ajout d'altérations connues, notamment de la réverbération ou de la distorsion.

L'invention vise également un procédé de commande vocale d'au moins un appareil électrique à l'aide d'un système de commande vocal relié audit appareil, lequel système de commande vocale comprend au moins un dispositif de commande vocale de l'appareil électrique, lequel procédé comprend au moins les étapes successives de :

- Détection d'un signal sonore dont au moins une partie émise par un utilisateur émetteur se situe dans une plage de fréquences dites vocales comprise entre 50 et 500 Hz ;
- Extraction d'un signal vocal à partir du signal sonore détecté et d'au moins une caractéristique représentative de ce signal vocal ;
- Comparaison de la caractéristique du signal vocal avec une base de données de caractéristiques représentatives de commandes vocales de référence compris dans un espace mémoire du dispositif de commande vocale et/ou un espace mémoire du serveur, chaque commande vocale correspondant à un signal logique de commande de l'appareil électrique, et
- Identification du signal logique de commande de l'appareil électrique.

Le procédé de commande peut également comporter les caractéristiques optionnelles suivantes considérées isolément ou selon toutes les combinaisons techniques possibles :

- Le dispositif de commande vocale est prévu pour commander au moins deux appareils électriques, le système de commande vocale comprenant un serveur relié au dispositif de commande vocale et qui comprend une base de données de caractéristiques de commandes vocales de référence associées aux appareils électriques considérés, lequel procédé comprend, suite à la génération de la commande vocale par le dispositif de commande vocale, les étapes successives de :

  - Envoi de la commande vocale vers le serveur ;
  - Comparaison par le serveur des caractéristiques de la commande vocale reçue avec les caractéristiques des commandes vocales de référence ;
  - Association de la commande vocale reçue avec la commande vocale de référence déterminée ;
  - Association de la commande vocale reçue avec l'appareil électrique à commander, et
  - Envoi au dispositif de commande vocale d'une information indiquant l'appareil électrique identifié devant être commandé.

L'invention concerne enfin une utilisation d'un système de commande tel que décrit précédemment pour mettre en œuvre un procédé d'avertissement tel que décrit précédemment afin d'avertir un soignant suite à l'émission d'un appel d'urgence par un patient ou un autre soignant, et/ou pour mettre en œuvre un procédé de commande vocale tel que décrit précédemment afin de permettre à un patient ou au soignant considéré de commander par la voix au moins un appareil électrique, et/ou pour dicter un texte enregistré dans un espace de stockage au moins du serveur, lequel système est apte à restituer vocalement le contenu du texte et fournir des instructions, lequel système est installé dans un établissement de soins médicaux ou paramédicaux.

D'autres caractéristiques et avantages de l'invention ressortiront clairement de la description qui en est donnée ci-dessous, à titre indicatif et nullement limitatif, en référence aux figures annexées :

[Fig 1] La figure 1 représente un schéma synoptique du système de commande vocale selon l'invention ;
[Fig 2] La figure 2 représente un schéma de l'antenne microphonique et du découpage de l'espace de réception de l'antenne en secteurs angulaires.

Il est tout d'abord précisé que sur les figures, les mêmes références désignent les mêmes éléments quelle que soit la figure sur laquelle elles apparaissent et quelle que soit la forme de représentation de ces éléments. De même, si des éléments ne sont pas spécifiquement référencés sur l'une des figures, leurs références peuvent être aisément retrouvées en se reportant à une autre figure.

Il est également précisé que les figures représentent essentiellement un mode de réalisation de l'objet de l'invention mais qu'il peut exister d'autres modes de réalisation qui répondent à la définition de l'invention.

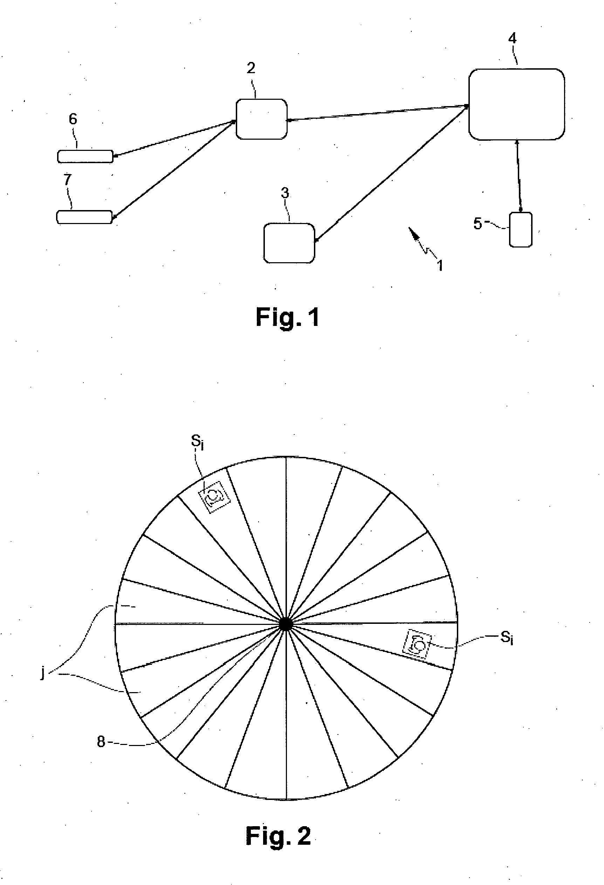

L'invention trouve son application dans le domaine de l'avertissement ou du contrôle d'appareils électriques par commandes vocales, à l'aide du système de commande vocale 1 de l'invention représenté sur la figure 1.

Selon l'invention, le système comprend au moins deux dispositifs de commande vocale 2, 3, et en particulier plusieurs dispositifs de commande vocale installés dans différentes pièces et/ou chambres d'un bâtiment. Chaque dispositif 2, 3 comprend des moyens d'acquisition d'un signal sonore, et en particulier une antenne microphonique 8 comprenant une pluralité de microphones. Avantageusement, l'antenne microphonique 8 est dite circulaire, c'est-à-dire que les microphones de l'antenne 8 sont arrangés de manière circonférentielle. Selon l'invention, l'antenne microphonique 8 comprend six microphones, sans que ce nombre ne soit limitatif dans le cadre de l'invention.

Les dispositifs de commande 2, 3 comprennent en outre des moyens d'émission d'un signal d'avertissement, et en particulier un signal d'avertissement sonore. A titre d'exemple, chaque dispositif 2, 3 peut comprendre une ou plusieurs enceintes, mais préférentiellement, l'antenne microphonique joue également le rôle d'émetteur sonore.

Enfin, chaque dispositif de commande 2, 3 comprend des moyens informatiques de traitement comprenant classiquement au moins un microprocesseur et un espace de stockage de données. Par ailleurs, les moyens informatiques comprennent des moyens de communication, préférentiellement des moyens de communications sans fil du type wifi ou Bluetooth.

Le système 1 comprend également un serveur informatique 4, le cas échéant relié à un réseau étendu du type internet. Ce serveur 4 comprend classiquement au moins un microprocesseur et un espace de stockage des données, et des moyens de communication, préférentiellement des moyens de communications sans fil du type wifi ou Bluetooth.

Les dispositifs de commande 2, 3 et le serveur 4 sont donc connectés ensemble au moins au sein d'un réseau local. Par ailleurs, le serveur 4 peut également être connecté, via un réseau étendu du type internet, à d'autres serveurs distants dits serveurs tiers.

Enfin, le système 1 comprend des moyens de détection et de localisation 5 d'au moins un utilisateur récepteur du signal d'avertissement, lesquels moyens de détection et de localisation 5 sont reliés au réseau local via des moyens de communication. Les moyens de détection et de localisation 5 comprennent par exemple une caméra, et peuvent comprendre un dispositif de géolocalisation ou simplement exploiter le réseau local wifi ou Bluetooth.

Avantageusement, les moyens de détection et de localisation 5 comprennent un dispositif d'identification des utilisateurs récepteurs. A titre d'exemple, chaque utilisateur récepteur comprend un badge émetteur comprenant des informations personnelles et en particulier son identité et sa fonction, tandis que les moyens de détection et de localisation 5 comprennent un dispositif récepteur apte à lire les informations contenues dans les badges émetteurs.

Le procédé d'avertissement par un signal sonore avec le système 1 de l'invention va maintenant être décrit.

Lorsqu'un premier dispositif 2 détecte un signal sonore dont au moins une partie est émise par un utilisateur émetteur et se situe dans une plage de fréquences comprise entre 50 Hz et 500 Hz, alors les moyens informatiques dudit dispositif 2 identifient au cours d'une première étape la présence d'un signal vocal dans le signal sonore. Ce signal vocal correspond à un appel émis par l'utilisateur émetteur qui est détecté par l'antenne microphonique du premier dispositif de commande 2. Le signal vocal est extrait par les moyens informatiques du premier dispositif 2 et en particulier au moins une caractéristique ou un ensemble de caractéristiques représentatives de ce signal vocal est extraite. A titre d'exemple, ces caractéristiques peuvent être l'amplitude ou encore la phase du signal vocal. Le signal vocal et sa ou ses caractéristiques sont envoyées vers le serveur informatique 4. Alternativement, le signal sonore est envoyé au serveur 4 dont les moyens informatiques extraient le signal vocal et la ou les caractéristiques considérées.

Les moyens informatiques du serveur 4 et des dispositifs de commande vocale 2, 3 comprennent une base de données associant la ou les caractéristiques considérées de chaque signal vocal à un signal d'avertissement sonore de référence. Alternativement, ces bases de données sont enregistrées sur des serveurs distants connectés au serveur 4 du système selon l'invention.

De fait, lorsque la caractéristiques du signal vocal est associé par les moyens informatiques considérés à un signal d'avertissement vocal (par exemple par un calcul de probabilité de proximité entre le signal vocal enregistré et des signaux vocaux de référence enregistrés dans la base de donnée du serveur 4), alors les moyens de détection et de localisation 5, au cours d'une deuxième étape, détectent et localisent un utilisateur récepteur dans le bâtiment, puis identifient au cours d'une troisième étape un second dispositif de commande 3 qui est le plus proche de l'utilisateur récepteur détecté et localisé.

De manière particulièrement avantageuse, la base de données de chaque dispositif 2, 3 et du serveur 4 ou des serveurs distants associe chaque signal d'avertissement sonore de référence à un utilisateur récepteur particulier ou le cas échéant à une fonction particulière. Ainsi, lorsqu'un signal d'avertissement sonore de référence est identifié, les moyens informatiques du serveur 4 associent ce signal avec le ou les utilisateurs récepteurs dont la fonction est la plus apte à résoudre l'appel de l'utilisateur émetteur. Puis les moyens de détection et de localisation 5 repèrent le dispositif de commande 3 qui est le plus proche de l'utilisateur récepteur apte parmi ceux détectés et localisés.

Bien entendu si aucun utilisateur récepteur, dont la fonction est associée au signal d'avertissement, n'est détecté, les moyens de détection et de localisation 5 chercheront l'un des utilisateurs récepteurs sans distinction de fonction.

Ainsi, une fois le second dispositif 3 le plus proche identifié, le serveur lui envoie au cours d'une quatrième étape le signal d'avertissement sonore identifié et ledit signal est émis par le second dispositif de commande vocale 3.

De manière alternative et sans sortir du cadre de l'invention, lorsque le signal vocal est associé au signal d'avertissement considéré, ce dernier est simplement émis sur un second dispositif de commande vocal 3 autre que le premier dispositif de commande 2 ayant capté le signal sonore. Ce second dispositif de commande vocal 3, émetteur du signal d'avertissement, est dans ce cas choisi aléatoirement ou par défaut par les moyens de traitement.

Par ailleurs, la fiabilité de la reconnaissance vocale mise en œuvre par le système de commande 1 de l'invention repose sur deux traitements de signaux sonores.

Pour cela, les moyens informatiques des dispositifs de commande vocale 2, 3 et du serveur 4 comprennent au moins deux algorithmes enregistrés dans les espaces de stockage considérés et programmés pour mettre en œuvre le procédé de l'invention. Les deux algorithmes comprennent des réseaux de neurones convolutifs, et préférentiellement des réseaux de neurones du type U-Net particulièrement adaptés à la segmentation de données.

Le premier traitement réalisé consécutivement par deux réseaux de neurones vise à séparer les sources d'émission sonore $s_i$, tandis que le second traitement réalisé par un troisième réseau de neurones vise à fiabiliser la reconnaissance des signaux sonores bruités. Avantageusement, le calcul de probabilité de proximité entre le signal vocal enregistré et des signaux vocaux de référence décrit ci-dessus peut-être réalisé par un des réseaux de neurones.

Les deux traitements sont mis en œuvre via le procédé de l'invention et vont maintenant être décrits.

Le premier traitement, comme indiqué ci-dessus, vise à identifier différentes sources sonores $s_i$, et en particulier différents signaux vocaux, et à sélectionner le signal vocal le plus proche du dispositif de commande considéré 2.

Pour cela, la zone de réception autour de l'antenne microphonique circulaire 8 est divisée en une pluralité de secteurs angulaires j de même angle, par exemple et de manière non limitative en 18 secteurs de 20 degrés. Les moyens de traitement du dispositif de commande 2 considèrent qu'une unique source sonore $s_i$ se trouve dans l'espace défini par un unique secteur j.

Par ailleurs, l'hypothèse « W disjoint orthogonalité » est considérée dans les calculs effectués par les moyens de traitement, ce qui signifie qu'il est admis qu'à un instant n et pour une fréquence f, une seule source sonore $s_i$ est active. Les moyens de traitement du dispositif de commande 2 estiment ainsi que le mélange sonore capté au niveau de chaque secteur j par l'antenne microphonique circulaire 8, pour une fréquence f déterminée et à un instant n déterminé, ne provient que d'une seule source $s_i$. On entend par mélange sonore le recouvrement des différents signaux sonores captés par l'antenne microphonique 8.

En outre, en plus de sa fréquence, un signal vocal est défini par son amplitude et par sa phase. Plus l'amplitude est forte et plus la valeur de la phase est élevée, et plus la source $s_i$ émettant le signal vocal est proche. Les moyens de traitement vont donc chercher le signal vocal présentant la plus forte amplitude et la phase la plus élevée pour identifier la source émettrice $s_i$ à prendre en considération pour in fine transmettre la commande vocale.

Les moyens de traitement du dispositif de commande 2, 3 ayant reçu le signal sonore vont appliquer, pour chaque secteur déterminé, un masque en fréquence et en temps sur le signal sonore reçu par l'antenne microphonique 8. A un instant n et pour une fréquence f déterminée, le masque pour chaque secteur j se présente selon l'équation suivante :

$$X_j(f, n) = \sum_{i=1}^{k} X_i(f, n)$$

Où $X_j(f, n)$ est le mélange sonore capté par le secteur j de l'antenne microphonique 8, et où Xi est le signal sonore perçu par le microphone i. Dans le cas présent où l'antenne microphonique comprend 6 micros, k = 6.

Par ailleurs; le signal sonore perçu par le microphone i s'exprime selon l'équation suivante : $X_i(f, n) = \gamma_i(f,n).s(f,n)$ , s(f, n) étant la source sonore captée par le microphone i à l'instant n pour la fréquence f et $\gamma_i$ un coefficient caractérisant l'amplitude et la phase du signal sonore capté par le microphone Xi.

Les moyens de traitement du dispositif de commande 2 vont par la suite exécuter le premier réseau de neurones afin d'appliquer pour chaque secteur j, pour chaque fréquence f et à chaque instant n, le masque Xj considéré et en déduire une estimation de l'amplitude et de la phase du signal perçu par le secteur j considéré. Dans le cas présent où la zone de réception circulaire de l'antenne microphonique 8 est divisée en 18 secteurs, j est un entier compris entre 1 et 18. 18 signaux sources (un par secteur j) sont ainsi estimés.

Pour encore améliorer la séparation des sources $s_i$, le second réseau de neurones est appliqué sur les signaux sources les plus significatifs, c'est-à-dire ceux dont l'amplitude est supérieure à un seuil déterminé.

Plus précisément, les moyens de traitement du dispositif de commande 2 réalisent une analyse spatiale du signal sonore par laquelle au moins deux signaux vocaux sont identifiés. Par la suite, les moyens de traitements déterminent une pluralité de directions couvrant le spectre spatial complet autour des .moyens d'acquisition du dispositif de commande vocale (2, 3). Puis, les moyens de traitements déterminent les caractéristiques spectrales du signal sonore associé à chaque direction, afin de déterminer les directions qui reçoivent des signaux sonores dont la puissance acoustique est supérieure à un seuil déterminé. Par la suite, les moyens de traitement associent chaque signal sonore dépassant le seuil déterminé à une source sonore ($s_i$), déterminent le nombre de sources sonores ($s_i$) et associent chaque source ($s_i$) à la direction considérée. Enfin, les caractéristiques spectrales de chaque source sonore identifiée ($s_i$) sont améliorées en en soustrayant à chacune de ces sources sonores ($s_i$) les caractéristiques spectrales des autres sources identifiées (si). Cette amélioration est également connue sous la dénomination de rehaussement.

Pour chaque signal sonore significatif sélectionné, trois grandeurs sont envoyées en entrée du second de réseau de neurones

La première grandeur est l'amplitude spectrale du signal sélectionné issu d'un secteur j estimé par un filtre connu, par exemple un filtre de Wien du type GEV beamformer.

La seconde grandeur est l'amplitude spectrale d'un signal sonore issu d'un microphone de référence préalablement et aléatoirement déterminé.

La troisième grandeur est la somme des amplitudes spectrales des autres signaux sonores sélectionnés (c'est-à-dire dont l'amplitude est supérieure au seuil déterminé) sur les autres secteurs j.

Au final, les moyens de traitement du dispositif de commande 2 identifient, parmi les signaux sonores dont l'amplitude est supérieure au seuil déterminé, celui qui présente la plus forte amplitude et la phase la plus grande. C'est sur ce signal sonore de plus forte amplitude que les étapes 1 à 4 du procédé décrites ci-dessus sont mises en œuvre.

Préalablement à la mise en œuvre du premier traitement de signaux, les coefficients $\gamma_i$ du premier réseau de neurones sont déterminés. Pour cela, le réseau de neurones est entraîné par rétropropagation avec des signaux sonores dont les caractéristiques sont connues et qui proviennent de sources sonores dont la position est également connue. Le second réseau de neurones est entraîné de la même façon.

Il est fréquent que le signal vocal émis par l'utilisateur émetteur et extrait du signal sonore par les moyens de traitement soit bruité, par exemple par de l'écho, des réverbérations ou subisse des distorsions. Pour encore améliorer la fiabilité du procédé de reconnaissance vocale, le troisième réseau de neurones est préalablement entraîné avec une base de données de caractéristiques correspondant à des altérations de signaux vocaux et dont la correspondance avec des signaux vocaux intacts est connue. De la sorte, le troisième réseau de neurones associe chaque caractéristique considérée du signal vocal altéré provenant de l'antenne microphonique avec la caractéristique correspondante d'un signal vocal intact. Plus précisément, chaque signal vocal altéré est associé avec un signal vocal intact de référence par comparaison de leurs caractéristiques et en associant les caractéristiques les plus proches. Par la suite, le troisième réseau de neurones identifie le signal vocal intact dans la base de données qui sera utilisé pour identifier le signal d'avertissement à transmettre.

Dans la mesure où un nombre important de données d'entrainement est nécessaire pour entrainer un réseau de neurones, un enrichissement des données d'entrainement est avantageusement réalisé préalablement à l'exécution du programme contenant le troisième réseau de neurones. Ces données enrichies sont générées en ajoutant des défauts et des altérations connues à des signaux vocaux non altérés, notamment de l'écho, des réverbérations ou des distorsions.

L'invention comprend également un procédé de commande d'au moins un appareil électrique 6, 7, qui va maintenant être décrit.

Le ou les appareils électriques 6, 7 sont reliés au réseau local afin d'être connectés au système de commande vocale 1 de l'invention.

De la même manière que le procédé d'avertissement décrit ci-dessus, le procédé de commande vocale d'un appareil électrique 6, 7 met en œuvre les mêmes deux premières étapes qui consistent à détecter un signal sonore et extraire un signal vocal dudit signal sonore et au moins une caractéristique. Bien entendu, les deux traitements de signaux mis en œuvre par les trois réseaux de neurones décrits ci-dessus sont également appliqués préalablement à la mise en œuvre de ces deux premières étapes.

La ou les caractéristiques du signal vocal extrait sont alors comparées avec une base de données de caractéristiques correspondant à des commandes vocales de référence comprise dans les espaces mémoire des dispositifs de commande vocale 2, 3 et du serveur 4, et le cas échéant des serveurs distants dits serveurs tiers, chaque commande vocale de référence correspondant à un signal logique de commande de l'appareil électrique considéré 6, 7.

En cas de présence d'un serveur tiers, le système va associer la commande vocale reçue avec une requête informatique d'interrogation de la base de données résidant sur le serveur tiers, puis de la part du serveur tiers la réponse à la requête informatique d'interrogation de la base de données, et enfin envoyer au dispositif de commande considéré 2, 3 les caractéristiques représentatives de la commande vocale de référence et associées à la réponse du serveur tiers.

Le signal logique de commande est alors identifié et envoyé à l'appareil considéré 6, 7.

Avantageusement, si plusieurs appareils électriques 6, 7 sont connectés dans la même pièce au système de commande vocal 2 de l'invention, chaque commande vocale de référence est également associée avec l'appareil considéré 6, 7. Les moyens de traitements effectuent donc deux étapes supplémentaires préalablement à l'identification du signal logique, et ces étape supplémentaires ' consistent d'une part à comparer la caractéristique de la commande vocale avec la base de données de caractéristiques représentatives des commandes vocales associées à plusieurs appareils électriques considérés 6, 7, et d'autre part à associer la commande vocale reçue avec l'appareil électrique à commander 6, 7, et à envoyer au dispositif de commande 2 considéré une information indiquant l'appareil électrique 6, 7 identifié devant être commandé.

Le système de commande vocale 1 s'applique à tous domaine où la commande par reconnaissance vocale est pertinente, mais le dit système 1 est particulièrement adapté pour une utilisation en milieu hospitalier, dans des établissements de soins médicaux ou paramédicaux, dans les établissements accueillant des personnes âgées, et plus généralement dans tous les environnements nécessitant une reconnaissance vocale fiable. Dans ce cas précis, les utilisateurs émetteurs sont des patients et les utilisateurs récepteurs sont des soignants.

Le système de commande 1 permet effectivement l'émission d'un appel d'urgence par un patient depuis un premier dispositif de commande 2, l'identification d'une commande d'urgence vocale à transmettre, l'identification et la localisation d'un soignant et l'émission de la commande vocale d'urgence par un second dispositif de commande 3 le plus proche du soignant identifié et localisé pour que ce dernier puisse intervenir le plus rapidement possible.

Bien entendu, le système 1 de l'invention permet également la transmission de commandes vocales entre soignants, ou encore permet au soignant de dicter un texte (par exemple rapport de visite en chambre, rapport d'intervention chirurgicale, rapport médical) qui sera ensuite enregistré dans l'espace de stockage du serveur 4 ou du serveur tiers. En retour, l'un des dispositifs de commande 2, 3 peut restituer vocalement le contenu du texte et fournir par exemple des instructions d'intervention, des rappels du statut de la dernière visite, etc...suite à une requête du type SQL (de l'anglais « Structured Query Language ») par le système 1 vers la base de données du serveur tiers.

## Revendications

1. Système de commande vocale (1) comprenant au moins :

   • Un premier dispositif de commande vocale (2) comportant des moyens d'acquisition d'un signal sonore et un second dispositif de commande vocale (3) comportant des moyens d'émission d'un signal d'avertissement, et
   • Un serveur (4) relié aux dispositifs de commande vocale,

   lequel système de commande vocale (1) est configuré pour:

   • Acquérir un signal sonore via le premier dispositif de commande vocale (2), au moins une partie dudit signal sonore émis par un utilisateur émetteur se situant dans une plage de fréquences dites vocales comprise entre 50 et 500 Hz, et
   • Emettre un signal d'avertissement à destination de l'utilisateur récepteur via le second dispositif de commande vocale (3).

2. Système (1) selon la revendication précédente, **caractérisé en ce qu'**il comprend des moyens de détection et de localisation (5) d'un utilisateur récepteur relié au serveur (4), et **en ce que** le système (1) est configuré pour :

   • Suite à l'acquisition du signal sonore, détecter et localiser un utilisateur récepteur via les moyens de détection et de localisation (5), et

   • Préalablement à l'émission du signal d'avertissement, identifier le second dispositif de commande vocale (3) le plus proche de la position de l'utilisateur récepteur détecté.

3. Système (1) selon la revendication précédente, **caractérisé en ce qu'**il comprend au moins un dispositif d'identification personnalisé prévu pour être associé à l'utilisateur récepteur considéré.

4. Système (1) selon l'une quelconque des revendications précédente, **caractérisé en ce que** chaque dispositif de commande vocale (2, 3) est apte à commander au moins un appareil électrique (6, 7) et comprend un espace de stockage comportant une base de données de caractéristiques représentatives de commandes vocales de référence, chaque commande vocale correspondant à un signal logique de commande de l'appareil électrique (6, 7), et des moyens informatiques aptes à :

   • Extraire un signal vocal à partir du signal sonore détecté ;
   • Extraire au moins une caractéristique du signal vocal ;
   • Comparer la caractéristique du signal vocal avec la base de données de caractéristiques représentatives de commandes vocales de référence, et
   • Identifier le signal logique de commande de l'appareil électrique (6, 7).

5. Système (1) selon la revendication précédente, **caractérisé en ce que** le serveur (4) comprend une base de données de caractéristiques représentatives de commandes vocales associées à plusieurs appareils électriques considérés (6, 7), lequel serveur (4) est apte à :

   • Comparer la caractéristique de la commande vocale avec la base de données de caractéristiques représentatives des commandes vocales associées à plusieurs appareils électriques considérés (6, 7) ;
   • Associer la commande vocale reçue avec l'appareil électrique à commander (6, 7), et
   • Envoyer au dispositif de commande vocale (2, 3) une information indiquant l'appareil électrique identifié (6, 7) devant être commandé.

6. Système selon l'une quelconque des revendications 1 à 5, **caractérisé en ce qu'**il comprend au moins un autre serveur dit serveur tiers, connecté au serveur (4) et comprenant une base de données de caractéristiques représentatives d'un signal vocal de référence, le système étant apte à :

   • Associer le signal vocal reçu avec une requête informatique d'interrogation de la base de données résidant sur le serveur tiers,

• Recevoir de la part du serveur tiers la réponse à la requête informatique d'interrogation de la base de données, et

• Envoyer au dispositif de commande vocale considéré (2, 3) les caractéristiques représentatives du signal vocal de référence associées à la réponse du serveur tiers.

7. Procédé d'avertissement par commande vocale à l'aide d'un système de commande vocale (1) comprenant au moins un premier dispositif de commande vocale (2) comportant des moyens de réception d'un signal sonore, un second dispositif de commande vocale (3) comportant des moyens d'émission d'un signal d'avertissement, des moyens de détection et de localisation (5) d'un utilisateur récepteur et un serveur (4) relié aux dispositifs de commande vocale (2, 3) et aux moyens de détection et de localisation (5), lequel procédé comprend au moins les étapes successives de :

• Détection par le premier dispositif de commande vocale (2) d'un signal sonore dont au moins une partie émise par un utilisateur émetteur se situe dans une plage de fréquences dites vocales comprise entre 50 et 500 Hz, et

• Emission d'un signal d'avertissement à destination de l'utilisateur récepteur via le second dispositif de commande vocale (3).

8. Procédé selon la revendication précédente, **caractérisé en ce qu'**il comprend en outre les étapes suivantes :

• Suite à l'étape de détection du signal sonore, détection et localisation d'un utilisateur récepteur, et

• Préalablement à l'étape d'émission du signal d'avertissement, identification du second dispositif de commande (3) le plus proche de la position de l'utilisateur récepteur détecté.

9. Procédé selon la revendication précédente, **caractérisé en ce que** le premier dispositif de commande vocale (2) et/ou le serveur (4) comprennent un espace de stockage comportant une base de données de caractéristiques de signaux vocaux de référence chacun correspondant à un signal d'avertissement vocal déterminé, lequel procédé comprend, préalablement à l'étape de détection et de localisation d'un utilisateur récepteur, les étapes successives de :

• Extraction d'un signal vocal à partir du signal sonore détecté et d'au moins une caractéristique représentative de ce signal vocal,

• Comparaison de la caractéristique du signal vocal avec la base de données de caractéristiques représentatives des signaux vocaux de référence, et

• Identification du signal d'avertissement vocal devant être émis par le second dispositif de commande vocale (3).

10. Procédé selon la revendication 6 ou 7, **caractérisé en ce qu'**une analyse spatiale du signal sonore identifie au moins deux signaux vocaux, et **en ce qu'**il comprend les étapes successives de :

• Détermination d'une pluralité de directions couvrant le spectre spatial complet autour des moyens d'acquisition du dispositif de commande vocale (2, 3),

• Détermination des caractéristiques spectrales du signal sonore associé à chaque direction,

• Détermination des directions recevant des signaux sonores dont la puissance acoustique est supérieure à un seuil déterminé,

• Association de chaque signal sonore dépassant le seuil déterminé à une source sonore (si),

• Détermination du nombre de sources sonores ($s_i$) et association de chaque source ($s_i$) à la direction considérée, et

• Amélioration des caractéristiques spectrales de chaque source sonore identifiée ($s_i$) en soustrayant à chacune de ces sources sonores ($s_i$) les caractéristiques spectrales des autres sources identifiées ($s_i$).

11. Procédé selon l'une quelconque des revendications précédentes, **caractérisé en ce qu'**il comprend une étape d'identification d'au moins une caractéristique correspondant à des altérations du signal vocal et d'association de chaque signal vocal altéré avec un signal vocal intact de référence correspondant par comparaison de leurs caractéristiques et en associant les caractéristiques les plus proches.

12. Procédé selon la revendication précédente, **caractérisé en ce qu'**il comprend une étape préalable d'enrichissement de la base de données de signaux altérés avec les signaux vocaux de référence modifiés par ajout d'altérations connues, notamment de la réverbération ou de la distorsion.

13. Procédé de commande vocale d'au moins un appareil électrique (6, 7) à l'aide d'un système de commande vocal (1) relié audit appareil (6, 7), lequel système de commande vocale (1) comprend au moins un dispositif de commande vocale (2, 3) de l'appareil électrique (6, 7), lequel procédé comprend au moins les étapes successives de :

• Détection d'un signal sonore dont au moins une partie émise par un utilisateur émetteur se situe dans une plage de fréquences dites vocales comprise entre 50 et 500 Hz ;

• Extraction d'un signal vocal à partir du signal sonore détecté et d'au moins une caractéristique représentative de ce signal vocal ;

• Comparaison de la caractéristique du signal vocal avec une base de données de caractéristiques représentatives de commandes vocales de référence compris dans un espace mémoire du dispositif de commande vocale (2, 3) et/ou un espace mémoire du serveur (4), chaque commande vocale correspondant à un signal logique de commande de l'appareil électrique (6, 7), et

• Identification du signal logique de commande de l'appareil électrique (6, 7).

14. Procédé selon la revendication précédente, **caractérisé en ce que** le dispositif de commande vocale (2, 3) est prévu pour commander au moins deux appareils électriques (6, 7), **en ce que** le système de commande vocale (1) comprend un serveur (4) relié au dispositif de commande vocale (2, 3) et qui comprend une base de données de caractéristiques de commandes vocales de référence associées aux appareils électriques considérés (6, 7), lequel procédé comprend, suite à la génération de la commande vocale par le dispositif de commande vocale (2, 3), les étapes successives de :

• Envoi de la commande vocale vers le serveur (4) ;

• Comparaison par le serveur (4) des caractéristiques de la commande vocale reçue avec les caractéristiques des commandes vocales de référence ;

• Association de la commande vocale reçue avec la commande vocale de référence déterminée ;

• Association de la commande vocale reçue avec l'appareil électrique à commander (6, 7), et

• Envoi au dispositif de commande vocale (2, 3) d'une information indiquant l'appareil électrique identifié (6, 7) devant être commandé.

15. Utilisation d'un système de commande (1) selon l'une quelconque des revendications 1 à 6 pour mettre en oeuvre un procédé d'avertissement selon l'une quelconque des revendications 7 à 12 afin d'avertir un soignant suite à l'émission d'un appel d'urgence par un patient ou un autre soignant, et/ou pour mettre en œuvre un procédé de commande vocale selon la revendication 13 ou 14 afin de permettre à un patient ou au soignant considéré de commander par la voix au moins un appareil électrique (6, 7), et/ou pour dicter un texte enregistré dans un espace de stockage au moins du serveur (4), lequel système (1) est apte à restituer vocalement le contenu du texte et fournir des instructions, lequel système (1) est installé dans un établissement de soins médicaux ou paramédicaux.

**Fig. 1**

**Fig. 2**

Europäisches
Patentamt

European
Patent Office

Office européen
des brevets

## RAPPORT DE RECHERCHE EUROPEENNE

Numéro de la demande

**EP 22 31 5055**

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| X | KR 2021 0155991 A (LG INNOTEK CO LTD [KR]) 24 décembre 2021 (2021-12-24) | 1-9,15 | INV. |
| Y | * alinéa [0008] * | 10-12 | G10L15/22 |
| | * alinéa [0020] * | | G10L21/0208 |
| | * alinéa [0023] * | | G10L21/0216 |
| | * alinéa [0026] * | | A61B5/00 |
| | * alinéas [0029] - [0030] * | | G16H80/00 |
| | * alinéas [0094] - [0100] * | | |
| | ----- | | |
| A | US 2021/240437 A1 (MAURY SEBASTIEN [FR] ET AL) 5 août 2021 (2021-08-05) | 4-6,9 | |
| | * alinéa [0036] * | | |
| | * alinéa [0126] * | | |
| | * alinéa [0157] * | | |
| | * alinéas [0159] - [0160] * | | |
| | * alinéa [0206] * | | |
| | * alinéas [0208] - [0209] * | | |
| | ----- | | |
| Y | US 10 755 728 B1 (AYRAPETIAN ROBERT [US] ET AL) 25 août 2020 (2020-08-25) | 10 | |
| | * colonne 3, lignes 58-67 * | | DOMAINES TECHNIQUES RECHERCHES (IPC) |
| | * colonne 5, lignes 62-67 * | | |
| | * colonne 6, lignes 39-67 * | | G10L |
| | * colonne 23, ligne 57 - colonne 24, ligne 14 * | | A61B |
| | | | G16H |
| | ----- | | |
| Y | US 2018/068675 A1 (VARIANI EHSAN [US] ET AL) 8 mars 2018 (2018-03-08) | 11,12 | |
| | * alinéas [0029] - [0030] * | | |
| | * alinéa [0048] * | | |
| | ----- | | |
| A | CN 110 960 750 A (UNIV YUNNAN) 7 avril 2020 (2020-04-07) | 15 | |
| | * alinéa [0025] * | | |
| | ----- | | |

~~Le présent rapport a été établi pour toutes les revendications~~

| Lieu de la recherche | Date d'achèvement de la recherche | Examinateur |
|---|---|---|
| La Haye | 26 août 2022 | Torcal Serrano, C |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

...............................................................
& : membre de la même famille, document correspondant

EPO FORM 1503 03.82 (P04C02)

Europäisches
Patentamt

European
Patent Office

Office européen
des brevets

**Numéro de la demande**

**EP 22 31 5055**

## REVENDICATIONS DONNANT LIEU AU PAIEMENT DE TAXES

La présente demande de brevet européen comportait lors de son dépôt les revendications dont le paiement était dû.

☐ Une partie seulement des taxes de revendication ayant été acquittée dans les délais prescrits, le présent rapport de recherche européenne a été établi pour les revendications pour lesquelles aucun paiement n'était dû ainsi que pour celles dont les taxes de revendication ont été acquittées, à savoir les revendication(s):

☐ Aucune taxe de revendication n'ayant été acquittée dans les délais prescrits, le présent rapport de recherche européenne a été établi pour les revendications pour lesquelles aucun paiement n'était dû.

## ABSENCE D'UNITE D'INVENTION

La division de la recherche estime que la présente demande de brevet européen ne satisfait pas à l'exigence relative à l'unité d'invention et concerne plusieurs inventions ou pluralités d'inventions, à savoir:

**voir feuille supplémentaire B**

☐ Toutes les nouvelles taxes de recherche ayant été acquittées dans les délais impartis, le présent rapport de recherche européenne a été établi pour toutes les revendications.

☐ Comme toutes les recherches portant sur les revendications qui s'y prêtaient ont pu être effectuées sans effort particulier justifiant une taxe additionnelle, la division de la recherche n'a sollicité le paiement d'aucune taxe de cette nature.

☐ Une partie seulement des nouvelles taxes de recherche ayant été acquittée dans les délais impartis, le présent rapport de recherche européenne a été établi pour les parties qui se rapportent aux inventions pour lesquelles les taxes de recherche ont été acquittées, à savoir les revendications:

☒ Aucune nouvelle taxe de recherche n'ayant été acquittée dans les délais impartis, le présent rapport de recherche européenne a été établi pour les parties de la demande de brevet européen qui se rapportent à l'invention mentionnée en premier lieu dans les revendications, à savoir les revendications:

**1-12(complètement); 15(en partie)**

☐ Le present rapport supplémentaire de recherche européenne a été établi pour les parties de la demande de brevet européen qui se rapportent a l'invention mentionée en premier lieu dans le revendications (Règle 164 (1) CBE)

Europäisches
Patentamt

European
Patent Office

Office européen
des brevets

**ABSENCE D'UNITÉ D'INVENTION**
**FEUILLE SUPPLÉMENTAIRE B**

Numéro de la demande

**EP 22 31 5055**

La division de la recherche estime que la présente demande de brevet européen ne satisfait pas à l'exigence relative à l'unité d'invention et concerne plusieurs inventions ou pluralités d'inventions, à savoir :

```
1. revendications: 1-12(complètement); 15(en partie)

   Système et procédé d'avertissement par commande vocale
   ---


2. revendications: 13, 14(complètement); 15(en partie)

   Procédé de commande vocale d'au moins un appareil électrique
   à l'aide d'un système de commande vocal relié audit appareil
   ---
```

EPO FORM P0402

## ANNEXE AU RAPPORT DE RECHERCHE EUROPEENNE
## RELATIF A LA DEMANDE DE BREVET EUROPEEN NO.

EP 22 31 5055

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

26-08-2022

| Document brevet cité au rapport de recherche | Date de publication | Membre(s) de la famille de brevet(s) | Date de publication |
|---|---|---|---|
| KR 20210155991 A | 24-12-2021 | AUCUN | |
| US 2021240437 A1 | 05-08-2021 | AUCUN | |
| US 10755728 B1 | 25-08-2020 | AUCUN | |
| US 2018068675 A1 | 08-03-2018 | US 2018068675 A1 | 08-03-2018 |
| | | US 2019259409 A1 | 22-08-2019 |
| | | US 2021295859 A1 | 23-09-2021 |
| CN 110960750 A | 07-04-2020 | AUCUN | |

EPO FORM P0460

Pour tout renseignement concernant cette annexe : voir Journal Officiel de l'Office européen des brevets, No.12/82